(19) Europäisches Patentamt
European Patent Office
Office européen des brevets

(11) Veröffentlichungsnummer: **0 098 972**
**A2**

(12) # EUROPÄISCHE PATENTANMELDUNG

(21) Anmeldenummer: 83105612.2

(22) Anmeldetag: 08.06.83

(51) Int. Cl.³: C 07 D 275/04
A 01 N 43/80

(30) Priorität: 19.06.82 DE 3222974

(43) Veröffentlichungstag der Anmeldung: 25.01.84 Patentblatt 84/4

(84) Benannte Vertragsstaaten: BE DE FR GB IT NL

(71) Anmelder: BASF Aktiengesellschaft
Carl-Bosch-Strasse 38
D-6700 Ludwigshafen(DE)

(72) Erfinder: Hagen, Helmut, Dr.
Max-Slevogt-Strasse 17e
D-6710 Frankenthal(DE)

(72) Erfinder: Ziegler, Hans, Dr.
Am Speyerweg 48
D-6704 Mutterstadt(DE)

(72) Erfinder: Wuerzer, Bruno, Dr. Dipl.-Landwirt
Ruedigerstrasse 13
D-6701 Otterstadt(DE)

(54) **Neue 5-Phenoxybenzisothiazol-4'-harnstoffderivate, Verfahren zu ihrer Herstellung und ihre Verwendung als Herbizide.**

(57) 5-Phenoxybenzisothiazol-4'-harnstoff der Formel I

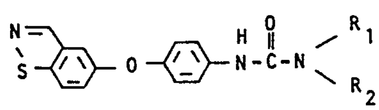

I

worin $R^1$ und $R^2$ einen gesättigten geradkettigen oder verzweigten aliphatischen Rest mit 1 bis 10 Kohlenstoffatomen, einen ungesättigten geradkettigen oder verzweigten aliphatischen Rest mit 3 bis 10 Kohlenstoffatomen, einen araliphatischen oder aromatischen Rest, der mit 1 bis 3 $C_1$-$C_4$-Alkyl-, $C_1$-$C_4$-Alkoxy-, Cyan-, Halogen-, Nitro-, Mono- oder Trifluoromethylgruppen substituiert sein kann, einen Alkoxyrest mit 1 bis 6 Kohlenstoffatomen, $R^2$ auch Wasserstoff oder $R^1$ und $R^2$ gemeinsam mit dem Stickstoffatom Glieder eines 5- oder 6-gliedrigen Ringes, der weiteren Stickstoff oder Sauerstoff als Heteroatom enthalten kann, und X Wasserstoff, Trifluoromethyl oder Chlor bedeuten, Verfahren zu dessen Herstellung sowie ein Herbizid, das den Harnstoff der Formel I als Wirkstoff enthält.

EP 0 098 972 A2

# Neue 5-Phenoxybenzisothiazol-4'-harnstoffderivate, Verfahren zu ihrer Herstellung und ihre Verwendung als Herbizide

Die Erfindung betrifft neue 5-Phenoxybenzisothiazol-4'-harnstoffderivate, Verfahren zu ihrer Herstellung und Herbizide, die diese Verbindungen als Wirkstoffe enthalten.

Naphthoxy- und Phenoxy-phenylharnstoffe mit herbiziden Eigenschaften sind bekannt (z.B. DE-OS 28 53 791, BE-PS 593 743, BE-PS 623 440, NL-OS 69 01 066, EP-OS 36 390).

Es kommt jedoch bei der Verwendbarkeit einer herbiziden Verbindung nicht nur auf die Stärke der Wirkung an, sondern in besonderem Maße auch auf die Selektivität gegenüber Nutzpflanzen. Solche Selektivitäten erfordern eine sehr spezifische chemische Struktur, die nicht aus Analogiebetrachtungen von bekannten Strukturen ableitbar ist.

Der Erfindung lag die Aufgabe zugrunde, Herbizide mit günstigeren Eigenschaften zu finden.

Die Lösung dieser Aufgabe besteht in den 5-Phenoxybenzisothiazol-4'-harnstoffen nach Anspruch 1.

Es wurde nämlich gefunden, daß 5-Phenoxybenzisothiazol-4'-harnstoffe der Formel I

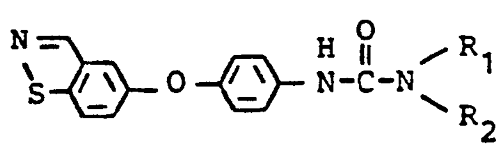

I

BR/P

worin $R^1$ und $R^2$ einen gesättigten geradkettigen oder verzweigten aliphatischen Rest mit 1 bis 10 Kohlenstoffatomen, einen ungesättigten geradkettigen oder verzweigten aliphatischen Rest mit 3 bis 10 Kohlenstoffatomen, einen araliphatischen oder aromatischen Rest, der mit 1 bis 3 $C_1$-$C_4$-Alkyl-, $C_1$-$C_4$-Alkoxy-, Cyan-, Halogen-, Nitro-, Mono- oder Trifluormethylgruppen substituiert sein kann, einen Alkoxyrest mit 1 bis 6 Kohlenstoffatomen, $R^2$ auch Wasserstoff oder $R^1$ und $R^2$ gemeinsam mit dem Stickstoffatom Glieder eines 5- oder 6-gliedrigen Ringes, der weiteren Stickstoff oder/und Sauerstoff als Heteroatom enthalten kann, und X Wasserstoff, Trifluormethyl oder Chlor bedeuten, eine selektive herbizide Wirkung zur Bekämpfung von unerwünschtem Pflanzenwuchs in Kulturpflanzen haben. Die neuen Verbindungen lassen sich herstellen, indem man

a) ein 5-Phenoxy-4'-aminobenzisothiazol der Formel II,

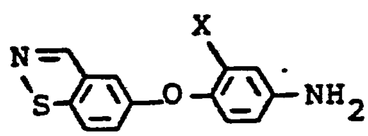

II

in der X die oben genannten Bedeutungen besitzt, in einem inerten Verdünnungsmittel mit einem Isocyanat der Formel III,

$$O{=}N{=}N{-}R^1 \qquad III$$

in der $R^1$ eine der oben genannten Bedeutungen hat, in Gegenwart eines Katalysators umsetzt.

Die Umsetzung kann durch folgende Reaktionsgleichung beschrieben werden:

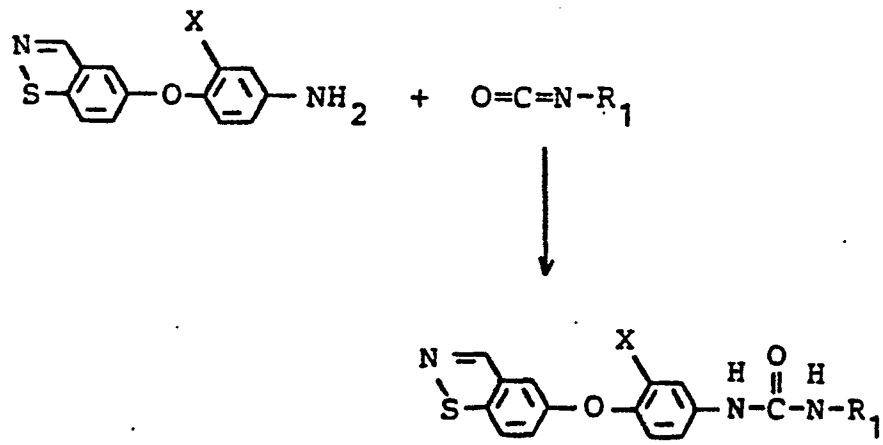

Für die Umsetzung kommen als inerte Lösungsmittel aliphatische oder aromatische Kohlenwasserstoffe, wie Benzin, Benzol oder Toluol, Carbonsäurenitrile wie Acetonitril, Ketone wie Aceton, chlorierte Kohlenwasserstoffe wie Dichlormethan, Chloroform, Tetrachlormethan, Ether und cyclische Ether wie Diethylether, Diisopropylether, Tetrahydrofuran und Dioxan in Frage. Bevorzugte Lösungsmittel sind Toluol und Dioxan. Als Katalysator wird eine inerte Base in einem Verhältnis von 1 bis 0,001 Teilen, vorzugsweise von 0,02 bis 0,05 Teilen, bezogen auf den Ausgangsstoff der Formel II, zugesetzt. Als Base wird Triethylamin bevorzugt.

Der Temperaturbereich der Umsetzung liegt bei 0 bis 200°C. Vorzugsweise wird die Umsetzung bei 60 bis 100°C ausgeführt.

Durch die Reaktion sind Verbindungen der Formel I, bei denen für $R^2$ Wasserstoff steht, herstellbar.

b) ein 5-Phenoxy-4'-isocyanatbenzisothiazol der Formel IV, in der X die oben genannten Bedeutungen hat,

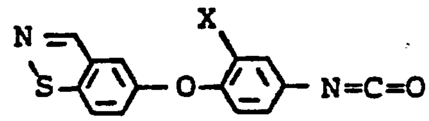

IV

in einem inerten Lösungsmittel mit einem Amin der Formel V, in der $R^1$ und $R^2$ die oben genannten Bedeutungen haben,

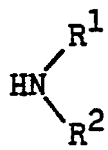

V

umsetzt.

Die Umsetzung kann durch die folgende Reaktionsgleichung beschrieben werden:

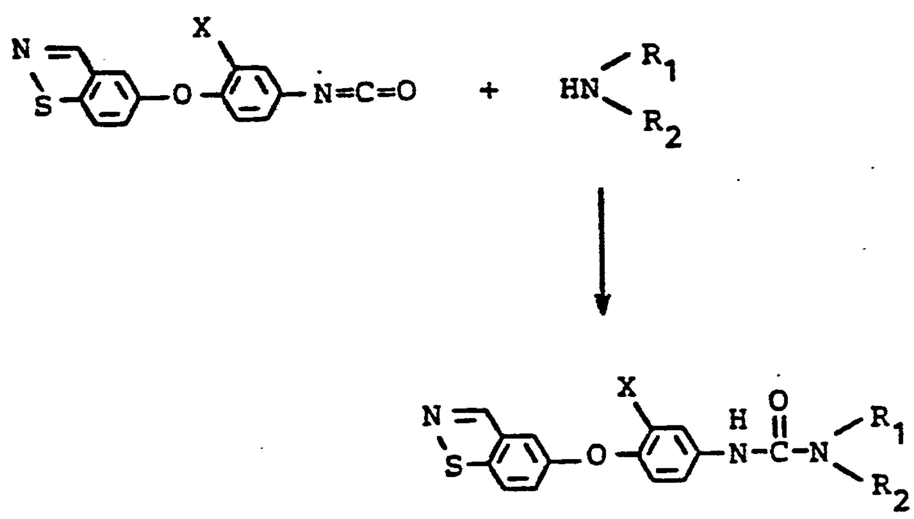

Für die Reaktion kommen die gleichen inerten Lösungsmittel in Frage wie die für die unter a) beschriebene Synthese, doch werden hier Acetonitril und Ether wie Diethylether, Tetrahydrofuran und Dioxan bevorzugt.

Der Temperaturbereich der Umsetzung liegt bei -70 bis 150°C, vorzugsweise bei 0 bis 30°C.

Von den Bedeutungen für $R^1$ und $R^2$ der Verbindungen der Formel I werden bevorzugt:

a) wenn X für Wasserstoff oder Chlor steht:
$R^1$ = Methyl, $R^2$ = Methyl
$R^1$ = Methyl, $R^2$ = Methoxy

b) wenn X für Trifluormethyl steht:
$R^1$ = Methyl, $R^2$ = Wasserstoff
$R^1$ = Methyl, $R^2$ = 1-Methylpropinyl
$R^1$ und $R^2$ gemeinsam mit dem N-Atom = 4-Morpholinyl

Herstellung

Die Herstellung der Ausgangsstoffe wird durch folgende Beispiele verdeutlicht. Teile beziehen sich auf das Gewicht.

Ausgangsstoff I
5-Phenoxy-4'-amino-benzisothiazol

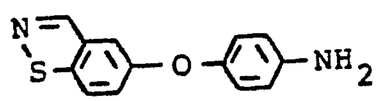

151 Teile 5-Hydroxybenzisothiazol, gelöst in 500 Teilen Methanol, wurden bei 20 bis 30°C mit 56 Teilen Kaliumhydroxid, gelöst in 30 Teilen Wasser, versetzt. Die Lösung

wurde im Vakuum zur Trockne eingeengt und der Rückstand in 850 Teilen Dimethylsulfoxid gelöst. In diese Lösung wurden 141 Teile 1-Fluor-4-nitrobenzol gegeben. Die Reaktionsmischung wurde 12 Stunden bei 80°C gerührt und anschließend mit 1000 Teilen Wasser versetzt und mit Dichlormethan extrahiert. Man erhielt 226 Teile 5-Phenoxy-4'-nitro-benzisothiazol entsprechend 83 % d. Theorie vom Schmp. 103°C.

109 Teile 5-Phenoxy-4'-nitro-benzisothiazol wurden in 600 Teilen Ethanol mit 3 Teilen eines Palladium/Aktivkohlekontaktes in Gegenwart von Wasserstoff in einer Hydrierapparatur bei 50°C geschüttelt, bis keine Wasserstoffaufnahme mehr erfolgte. Nach Entfernen des Kontaktes und Einengen wurden 85 Teile 5-Phenoxy-4'-amino-benzisothiazol entsprechend 88 % d. Theorie vom Schmp. 100°C erhalten.

<u>Ausgangsstoff 2</u>

5-Phenoxy-4'-isocyanato-benzisothiazol

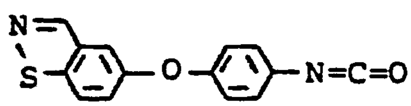

In 60,5 Teil 5-Phenoxy-4'-aminobenzisothiazol, gelöst in 400 Teilen Chlorbenzol wurden bei -5 bis -10°C 58 Teile Phosgen eingeleitet. Die Reaktionsmischung wurde 1 Stunde bei 20 bis 30°C gerührt und anschließend langsam auf 100°C gebracht. Nach Entfernen des Lösungsmittels und des überschüssigen Phosgens im Vakuum erhielt man 65 Teile 5-Phenoxy-4'-isocyanato-benzisothiazol entsprechend 97 % d. Theorie als helles Öl, das direkt zur Herstellung der Verbindungen der Formel I verwendet wurde.

Ausgangsstoff 3

5-Phenoxy-4'-amino-2'-chlor-benzisothiazol

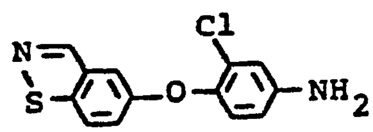

151 Teile 5-Hydroxybenzisothiazol wurden analog wie bei Ausgangsstoff 1 beschrieben mit 192 Teilen 3,4-Dichlor--nitrobenzol umgesetzt. Man erhielt 240 Teile 5-Phenoxy-2'--chlor-4'-nitro-benzisothiazol entsprechend 78 % d. Theorie vom Schmp. 150°C.

61,3 Teile 5-Phenoxy-2'-chlor-4'-nitro-benzisothiazol wurden analog Ausgangsstoff 1 reduziert. Man erhielt 55 Teile entsprechend 99 % d. Theorie 5-Phenoxy-4'-amino-2'-chlor--benzisothiazol.

Ausgangsstoff 4

5-Phenoxy-2'-chlor-4'-isocyanato-benzisothiazol

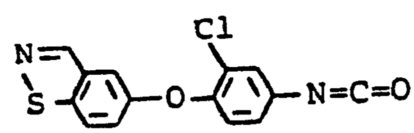

55,3 Teile 5-Phenoxy-4'-amino-2'-chlor-benzisothiazol wurden analog Ausgangsstoff 2 mit Phosgen umgesetzt. Man erhielt 60 Teile 5-Phenoxy-2'-chlor-4'-isocyanato-benz-isothiazol entsprechend 99 % d. Theorie als helles Öl.

Ausgangsstoff 5

5-Phenoxy-4'-amino-2'-trifluormethyl-benzisothiazol

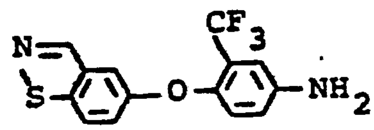

120 Teile 5-Hydroxybenzisothiazol wurden analog Ausgangsstoff 1 mit 176 Teilen 2-Chlor-5-nitro-benzotrifluorid umgesetzt. Man erhielt 253 Teile 5-Phenoxy-4'-nitro-2'-trifluormethyl-benzisothiazol entsprechend 93 % d. Theorie vom Schmp. 124°C.

136 Teile 5-Phenoxy-4'-nitro-2'-trifluormethyl-benzisothiazol wurden analog Ausgangsstoff 2 mit Wasserstoff reduziert. Man erhielt 110 Teile 5-Phenoxy-4'-amino-2'-trifluormethyl-benzisothiazol entsprechend 89 % d. Theorie vom Schmp. 136°C.

Ausgangsstoff 6

5-Phenoxy-4'-isocyanato-2'-trifluormethyl-benzisothiazol

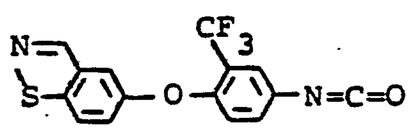

124 Teile 5-Phenoxy-4'-amino-2'-trifluormethyl-benzisothiazol wurden analog Ausgangsstoff 2 mit 70 Teilen Phosgen umgesetzt. Man erhielt 130 Teile 5-Phenoxy-4'-isocyanato-2'-trifluormethyl-benzisothiazol entsprechend 97 % d. Theorie als helles Öl.

Beispiel 1

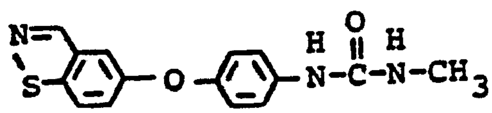

5-Phenoxy-4'-methylaminocarbonylamino-benzisothiazol

24,2 Teile 5-Phenoxy-4'-amino-benzisothiazol wurden mit 400 Teilen Toluol, 1 Teil Triethylamin und 11,4 Teilen Methylisocyanat 12 Stunden auf 80°C erwärmt. Die Reaktionsmischung wurde im Vakuum eingeengt. Man erhielt 25 Teile 5-Phenoxy-4'-methylaminocarbonylamin-benzisothiazol entsprechen 85 % d. Theorie vom Schmp. 178°C.

Beispiel 2

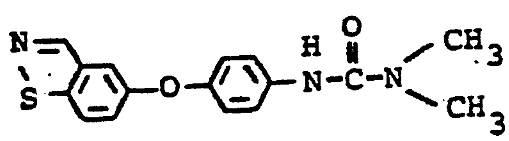

5-Phenoxy-4'-dimethylaminocarbonylamino-benzisothiazol

In eine Lösung von 26,8 Teilen 5-Phenoxy-4'-isocyanatobenzisothiazol in 300 Teilen Diethylether wurden 5 Teile Dimethylamin gasförmig eingeleitet und die Reaktionsmischung 12 Stunden bei 20 bis 30°C gerührt. Der ausgefallene Niederschlag wurde abgetrennt. Man erhielt 27,6 Teile 5-Phenoxy-4'-dimethylaminocarbonylamino-benzisothiazol entsprechend 88 % d. Theorie vom Schmp. 156°C.

Beispiel 3

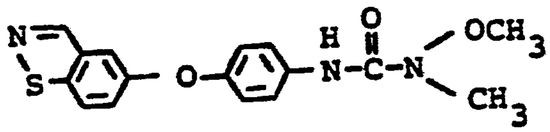

5-Phenoxy-4'-N-methoxy-N-methylaminocarbonylamino-benzisothiazol

26,8 Teile 5-Phenoxy-4'-isocyanato-benzisothiazol, in 300 Teilen Diethylether gelöst, wurden bei 20 bis 30°C mit 6,1 Teilen N-Methoxy-N-methylamin versetzt und 12 Stunden bei 20 bis 30°C gerührt. Anschließend wurde eingeengt und der Rückstand in ca. 50 Teilen Diethylether zerrieben, abgetrennt und getrocknet. Man erhielt 26 g 5-Phenoxy-4'-N--methoxy-N-methylaminocarbonylamino-benzisothiazol entsprechend 82 % d. Theorie vom Schmp. 108°C.

Analog Beispiel 2 wurden die Verbindungen der Beispiele 4 bis 10 hergestellt.

Beispiel 4

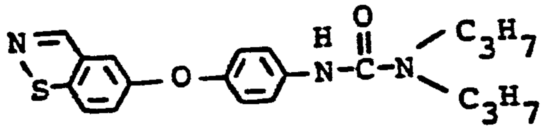

5-Phenoxy-4'-dipropylaminocarbonylamino-benzisothiazol Schmp. 113°C.

Beispiel 5

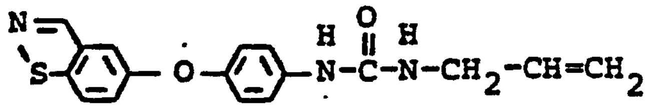

5-Phenoxy-4'-allylaminocarbonylamino-benzisothiazol
Schmp. 98°C.

Beispiel 6

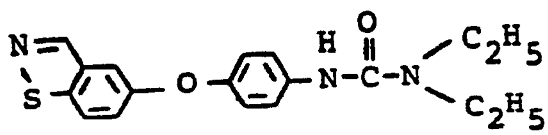

5-Phenoxy-4'-diethylaminocarbonylamino-benzisothiazol
Schmp. 147°C.

Beispiel 7

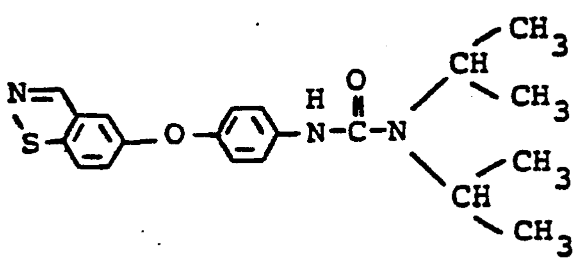

5-Phenoxy-4'-diisopropylaminocarbonylamino-benzisothiazol
Schmp. 137°C.

Beispiel 8

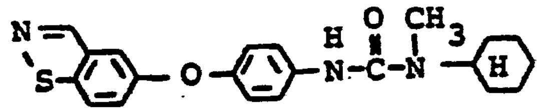

5-Phenoxy-4'-N-methyl-N-cyclohexylaminocarbonylamino-benzisothiazol

Beispiel 9

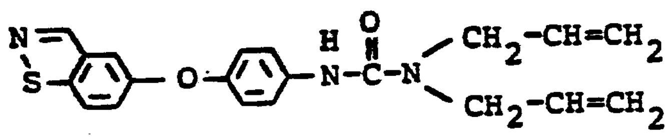

5-Phenoxy-4'-diallylaminocarbonylamino-benzisothiazol Schmp. 113°C.

Beispiel 10

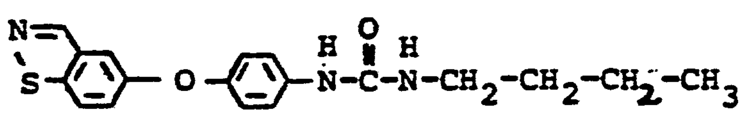

5-Phenoxy-4'-n-butylaminocarbonylamino-benzisothiazol Schmp. 120°C.

Beispiel 11

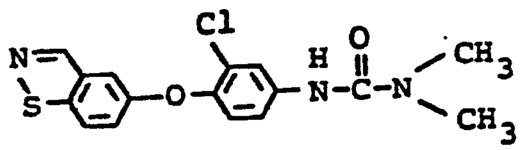

5-Phenoxy-(2'-chlor-4'-dimethylaminocarbonylamino)-benzisothiazol

In 30,3 Teilen 5-Phenoxy-(2'-chlor-4'-isocyanato)-benzisothiazol und 300 Teilen Diethylether wurden 5 Teile Dimethylamin bei 20 bis 30°C gasförmig eingeleitet. Nach 12-stündigem Rühren bei 20 bis 30°C wurde der ausgefallene Niederschlag abgetrennt und getrocknet. Man erhielt 27,5 Teile 5-Phenoxy-(2'-chlor-4'-dimethylamino-carbonylamino)-benzisothiazol entsprechend 70 % d. Theorie vom Schmp. 149°C.

Beispiel 12

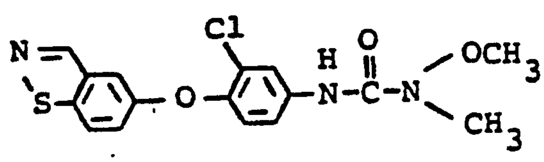

5-Phenoxy-[2'-chlor-4'-(N-methoxy-N-methylamino-carbonylamino)]-benzisothiazol

30,3 Teile 5-Phenoxy-(2'-chlor-4'-isocyanato)-benzisothiazol gelöst in 300 Teilen Diethylether wurden bei 20 bis 30°C mit 6,1 Teilen N-Methoxymethylamin, gelöst in 100 Teilen Diethylether, versetzt. Nach 12 Stunden bei 20 bis 30°C wurde die Reaktionsmischung eingeengt und der Rückstand mit ca. 50 Teilen Diethylether behandelt. Man

erhielt 30,2 Teile 5-Phenoxy-ß2'-chlor-4'-(N-methoxy-N--methylamino-carbonylamino)]-benzisothiazol entsprechend 83 % d. Theorie vom Schmp. 128°C.

Analog Beispiel 12 wurde

Beispiel 13

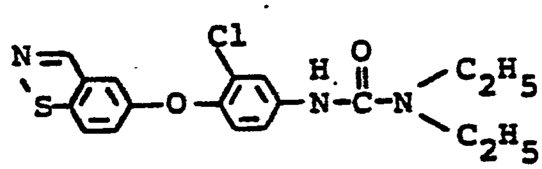

5-Phenoxy-(2'-chlor-4'-diethylaminocarbonylamino)-benzisothiazol
Schmp. 141°C
hergestellt.

Beispiel 14

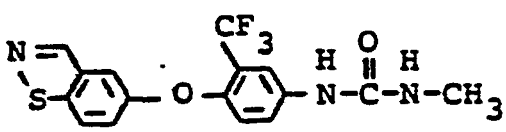

5-Phenoxy-(4'-methylaminocarbonylamino-2'-trifluormethyl)--benzisothiazol

31 Teile 5-Phenoxy-(4'-amino-2'-trifluormethyl)-benzisothiazol wurden mit 400 Teilen Toluol, 1 Teil Triethylamin und 11,4 Teilen Methylisocyanat 12 Stunden bei 80°C gerührt. Danach wurde das Lösungsmittel im Vakuum entfernt und der Rückstand über Kieselgel mit Chloroform als Laufmittel gereinigt.

Man erhielt 29,4 Teile 5-Phenoxy-(4'-methylamino-carbonylamino-2'-trifluormethyl)-benzisothiazol entsprechend 80 % d. Theorie vom Schmp. 138°C.

Analog Beispiel 14 wurde

Beispiel 15

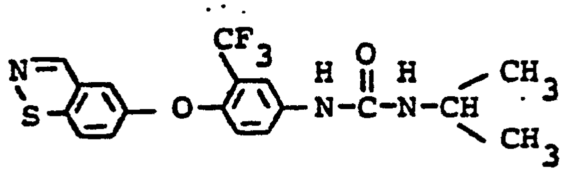

5-Phenoxy-(4'-isopropylaminocarbonylamino-2'-trifluormethyl)-benzisothiazol
Schmp. 158°C
hergestellt.

Beispiel 16

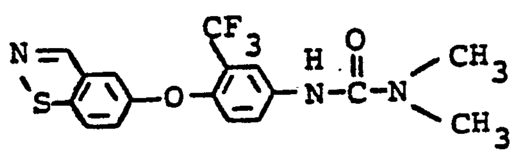

5-Phenoxy-(4'-dimethylaminocarbonylamino-2'-trifluormethyl)-benzisothiazol

In 33,6 Teile 5-Phenoxy-(4'-isocyanato-2'-trifluormethyl)-benzisothiazol, gelöst in 400 Teilen Acetonitril, wurden bei 10 bis 20°C 5 Teile Dimethylamin gasförmig eingeleitet. Nach 12-stündiger Reaktionszeit bei 20 bis 30°C wurde das Lösungsmittel im Vakuum entfernt. Aus Chloroform erhielt man 32,7 Teile 5-Phenoxy-(4'-dimethylaminocarbonyl-

amino-2'-trifluormethyl)-benzisothiazol entsprechend 86 % d. Theorie vom Schmp. 188°C.

Beispiel 17

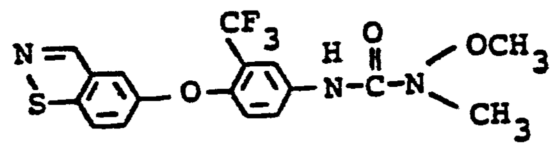

5-Phenoxy-[4'-(N-methoxy-N-methylaminocarbonylamino)-2'--trifluormethyl]-benzisothiazol

33,6 Teile 5-Phenoxy-(4'-isocyanato-2'-trifluormethyl)--benzisothiazol, gelöst in 300 Teilen Diethylether, wurden bei 20 bis 30°C mit 6,1 Teilen N-Methoxy-N-methylamin, gelöst in 100 Teilen Diethylether, versetzt. Die Reaktionsmischung wurde 12 Stunden bei 20 bis 30°C gerührt und anschließend der ausgefallene Niederschlag abgetrennt.

Man erhielt 33 Teile 5-Phenoxy-[4'-(N-methoxy-N-methylaminocarbonylamino)-2'-trifluormethyl]-benzisothiazol entsprechend 83 % d. Theorie vom Schmp. 197°C.

Analog Beispiel 17 wurden die Verbindungen der Beispiele 18 bis 24 hergestellt.

Beispiel 18

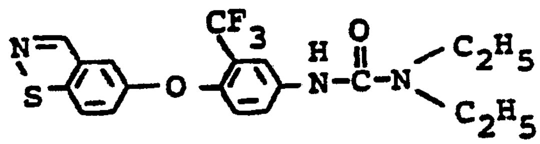

5-Phenoxy-(4-diethylaminocarbonylamino-2'-trifluormethyl)--benzisothiazol
Schmp. 156°C.

Beispiel 19

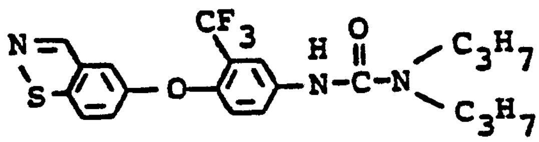

5-Phenoxy-[4'-n-dipropylaminocarbonylamino-2'-trifluormethyl]-benzisothiazol
Schmp. 129°C.

Beispiel 20

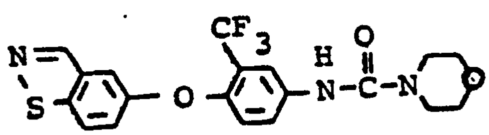

5-Phenoxy-(4'-morpholin-4-yl-carbonylamino-2'-trifluormethyl)-benzisothiazol
Schmp. Öl

Beispiel 21

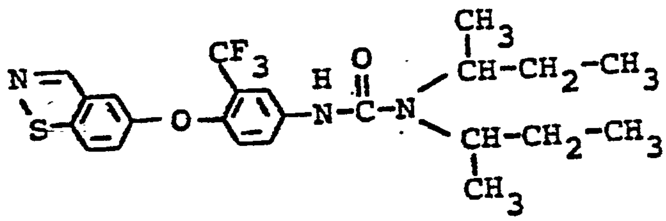

5-Phenoxy-[4'-(di-sec.-butyl)-aminocarbonylamino-2'-trifluormethyl]-benzisothiazol
Schmp. 100°C.

Beispiel 22

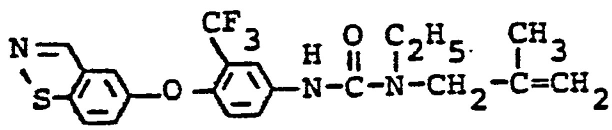

5-Phenoxy-[4'-(N-ethyl-N-methallylaminocarbonylamino)-2'-trifluormethyl]-benzisothiazol
Schmp. Öl

Beispiel 23

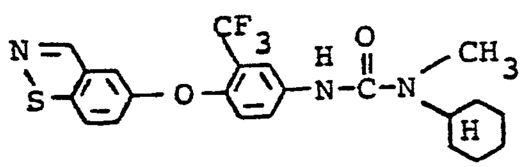

5-Phenoxy-[4'-(N-methyl-N-cyclohexylaminocarbonylamino)-2'-trifluormethyl]-benzisothiazol
Schmp. 147°C.

Beispiel 24

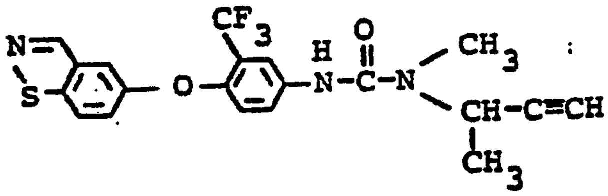

5-Phenoxy-[4'-(N-methyl-N-1-methylpropin-1-ylaminocarbonylamino)-2'-trifluormethyl]-benzisothiazol
Schmp. Öl

Anwendung

Die Anwendung der Wirkstoffe erfolgt z.B. in Form von direkt versprühbaren Lösungen, Pulvern, Suspensionen - auch hochprozentigen wäßrigen, öligen oder sonstigen Suspensionen - oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenglykolether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gewichtsprozent Wirkstoff, vorzugsweise zwischen 0,5 und 90 Gewichtsprozent.

Die Wirkstoffe werden angewendet beispielsweise durch Gießen, Streuen, Stäuben, Spritzen oder Sprühen auf die Pflanzen oder den Boden, durch Injizieren oder Bestreichen von Pflanzen oder durch Einbringen in das Bewässerungswasser.

Die Applikation der Mittel kann im Vorauflaufverfahren und bei Nachauflaufanwendung erfolgen. Vorzugsweise werden die neuen Wirkstoffe nach dem Auflaufen der unerwünschten Pflanzen ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post--directed, lay-by).

Die Aufwandmengen an Wirkstoff betragen je nach Bekämpfungsziel, Jahreszeit, Zielpflanzen und Wachstumsstadien derselben 0,1 bis 5 kg Wirkstoff/ha.

Der Einfluß von Vertretern der neuen 5-Phenoxybenzisothiazol-4'-harnstoffderivate auf das Wachstum von erwünschten und unerwünschten Pflanzen wird anhand von Gewächshausversuchen aufgezeigt:

Als Kulturgefäße dienten Plastikblumentöpfe mit 300 $cm^3$ Inhalt und lehmigem Sand mit etwa 1,5 % Humus als Substrat. Für die hier beschriebene Nachauflaufbehandlung zog man die Testpflanzen je nach Wuchsform bis zu einer Wuchshöhe von 3 bis 15 cm an und behandelte sie danach. Die Soja zog man in einem mit Torfmull (peat) angereicherten Substrat an, um ein günstigeres Wachstum zu gewährleisten. Zur Nachauflaufbehandlung wurden entweder direkt gesäte und in den gleichen Gefäßen aufgewachsene Pflanzen ausgewählt, oder aber sie wurden erst als Keimpflanzen getrennt angezogen und einige Tage vor der Behandlung in die Versuchsgefäße verpflanzt. Für das Aufbringen der Mittel wurden diese in Wasser als Verteilungsmittel suspendiert oder emulgiert und mittels fein verteilender Düsen gespritzt. Die Aufwandmengen variierten je nach Wirkstoff. Sie betrugen 0,5, 2,0 und 3,0 kg Wirkstoff/ha.

In die Versuche waren folgende Testpflanzen einbezogen:

| Botanischer Name | Deutscher Name | Englischer Name |
|---|---|---|
| Amaranthus spp. | Fuchsschwanzarten | pigweed |
| Avena sativa | Hafer | oats |
| Cassia tora | - | sicklepod |
| Centaurea cyanus | Kornblume | cornflower |
| Chenopodium album | Weißer Gänsefuß | lambsquarters |
| Glycine max. | Soja | soybeans |
| Ipomoea spp. | Prunkwindearten | morningglory |
| Sinapis alba | Weißer Senf | white mustard |
| Triticum aestivum | Weizen | wheat |

Die Versuchsgefäße wurden im Gewächshaus aufgestellt, wobei für wärmeliebende Arten wärmere Bereiche (20 bis 35°C) und für solche gemäßigter Klimate 10 bis 20°C bevorzugt wurden. Die Vergleichsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Aufgang und 100 kein Aufgang bzw. völlige Zerstörung zumindest der oberirdischen Sproßteile.

Die Gewächshausversuche zeigen die herbizide Wirksamkeit der Verbindungen Nr. 2, 3 und 14 (hergestellt gemäß Beispiel 2, 3 und 14) bei Nachauflaufanwendung von 3,0 kg Wirkstoff/ha. Sie zeigen weiterhin eine solche beispielsweise für die Verbindungen Nr. 20 und 24 bei Nachauflaufanwendung von 2,0 kg/ha.

Die selektive herbizide Wirkung bringen Beispiele mit der Verbindung Nr. 14 bei Nachauflaufanwendung von 0,5 kg Wirkstoff/ha zu Soja und Weizen und ebenso mit der Verbindung Nr. 11 bei Nachauflaufanwendung von 3,0 kg/ha zu Hafer.

In Anbetracht der guten Verträglichkeit für zahlreiche breitblättrige und andere Kulturen und der Vielseitigkeit der Applikationsmethoden können die erfindungsgemäßen Herbizide oder diese enthaltende Mittel noch in einer weiteren großen Zahl von Kulturpflanzen zur Beseitigung unerwünschten Pflanzenwuchses eingesetzt werden.

In Betracht kommen beispielsweise folgende Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor - Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |

| Botanischer Name | Deutscher Name | Englischer Name |
|---|---|---|
| Cucumis sativus | Gurke | cucumber |
| Cynodon dactylon | Bermudagras | Bermudagrass in turfs and lawn |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |

| Botanischer Name | Deutscher Name | Englischer Name |
|---|---|---|
| Manihot esculenta | Maniok | cassava |
| Medicago sativa | Luzerne | alfalfa (lucerne) |
| Metha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |

| Botanischer Name | Deutscher Name | Englischer Name |
|---|---|---|
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn maize |

Zur Verbreiterung des Wirkungsspektrums und zur Erzielung auch synergistischer Effekte können die neuen 5-Phenoxybenzisothiazol-4'-harnstoffderivate mit zahlreichen anderen herbiziden Wirkstoffen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, andere Harnstoffe, Diphenylether, Triazinone, Uracile, Benzofuranderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Tabelle 1 - Herbizide Aktivität von Beispielsverbindungen bei Nachauflaufanwendung von 3,0 kg/ha im Gewächshaus

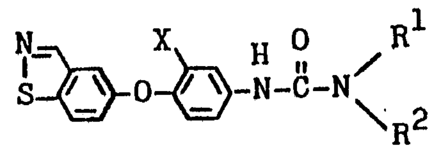

| Wirkstoff Nr. | X | $R^1$ | $R^2$ | Testpflanzen und Schädigung % Amaranthus spp. | Ipomoea spp. | Centaurea cyanus |
|---|---|---|---|---|---|---|
| 14 | $CF_3$ | H | $CH_3$ | 100 | 100 | 90 |
| 3 | H | $CH_3$ | $OCH_3$ | 100 | 100 | 100 |
| 2 | H | $CH_3$ | $CH_3$ | 100 | 70 | 100 |

Tabelle 2 - Herbizide Aktivität bei Nachauflaufanwendung von 2,0 kg/ha im Gewächshaus am Beispiel der breitblättrigen Testpflanze Sinapis alba

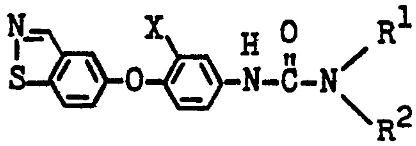

| Wirkstoff Nr. | X | $R^1$ | $R^2$ | Schädigung in % |
|---|---|---|---|---|
| 20 | $CF_3$ | 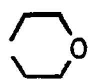 | | 90 |
| 24 | $CF_3$ | $-CH_3$ | $-\underset{CH_3}{CH}-C\equiv CH$ | 95 |

Tabelle 3 - Selektive Bekämpfung von Chenopodium album bei Nachauflaufanwendung von Wirkstoff Nr. 14 im Gewächshaus

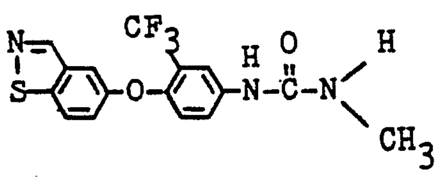

| Testpflanzen | Schädigung % bei 0,5 kg/ha |
|---|---|
| Glycine max. | 5 |
| Triticum aestivum | 3 |
| Chenopodium album | 90 |

Tabelle 4 - Selektive Bekämpfung von unerwünschten breitblättigen Pflanzen in Hafer mit Wirkstoff Nr.11 bei Nachauflaufanwendung von 3,0 kg/ha im Gewächshaus

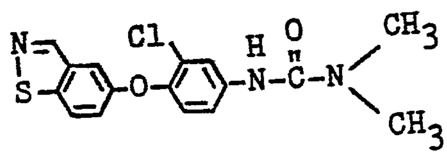

| Testpflanzen | Schädigung % |
|---|---|
| Avena sativa | 0 |
| Amaranthus spp. | 90 |
| Ipomoea spp. | 100 |
| Centaurea cyanus | 100 |
| Cassia tora | 100 |

Patentansprüche

1. 5-Phenoxybenzisothiazol-4'-harnstoff der Formel I

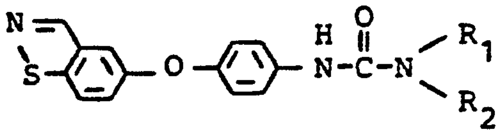

I

worin $R^1$ und $R^2$ einen gesättigten geradkettigen oder verzweigten aliphatischen Rest mit 1 bis 10 Kohlenstoffatomen, einen ungesättigten geradkettigen oder verzweigten aliphatischen Rest mit 3 bis 10 Kohlenstoffatomen, einen araliphatischen oder aromatischen Rest, der mit 1 bis 3 $C_1$-$C_4$-Alkyl-, $C_1$-$C_4$-Alkoxy-, Cyan-, Halogen-, Nitro-, Mono- oder Trifluormethylgruppen substituiert sein kann, einen Alkoxyrest mit 1 bis 6 Kohlenstoffatomen, $R^2$ auch Wasserstoff oder $R^1$ und $R^2$ gemeinsam mit dem Stickstoffatom Glieder eines 5- oder 6-gliedrigen Ringes, der weiteren Stickstoff oder Sauerstoff als Heteroatom enthalten kann, und X Wasserstoff, Trifluormethyl oder Chlor bedeuten.

2. Verfahren zur Herstellung von 5-Phenoxybenzisothiazol-4'-harnstoffen der Formel I, dadurch gekennzeichnet, daß man entweder

a) ein 5-Phenoxy-4'-aminobenzisothiazol der Formel II,

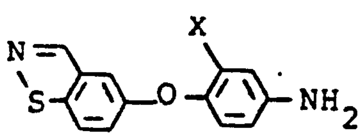

II

in der X die oben genannten Bedeutungen hat, mit einem Isocyanat der Formel III,

$$O{=}C{=}N{-}R^1 \qquad III$$

in der $R^1$ die oben genannten Bedeutungen hat, umsetzt, oder

b) ein 5-Phenoxy-4'-isocyanatobenzisothiazol der Formel IV

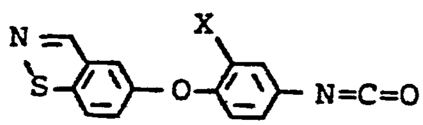

IV

in der X eine der oben genannten Bedeutungen hat, mit einem Amin der Formel V

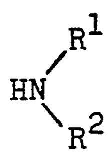

V

in der $R^1$ und $R^2$ die oben genannten Bedeutungen haben, in Gegenwart eines inerten Lösungsmittels umsetzt.

3. Herbizid, das einen festen oder flüssigen Trägerstoff und mindestens eine Verbindung gemäß Anspruch 1 als Wirkstoff in wirksamer Menge enthält.